**Europäisches Patentamt**

**European Patent Office**

**Office européen des brevets**

(19)

(11) Veröffentlichungsnummer: **0 081 185**
**B1**

(12) **EUROPÄISCHE PATENTSCHRIFT**

(45) Veröffentlichungstag der Patentschrift:
29.08.84

(21) Anmeldenummer: **82111060.8**

(22) Anmeldetag: **30.11.82**

(51) Int. Cl.³: **C 12 P 19/24**

(54) Verfahren und Anlage zur Herstellung von Isomerose.

(30) Priorität: **09.12.81 DE 3148603**

(43) Veröffentlichungstag der Anmeldung:
**15.06.83 Patentblatt 83/24**

(45) Bekanntmachung des Hinweises auf die Patenterteilung:
**29.08.84 Patentblatt 84/35**

(84) Benannte Vertragsstaaten:
**AT BE CH DE FR GB IT LI LU NL SE**

(56) Entgegenhaltungen:
**EP - A - 0 000 028**
**WO - A - 81/01418**
**DE - A - 2 715 041**
**US - A - 4 014 711**

(73) Patentinhaber: **Kali-Chemie Aktiengesellschaft,
Postfach 220 Hans-Böckler-Allee 20,
D-3000 Hannover 1 (DE)**

(72) Erfinder: **Weidenbach, Günter, Dr. Dipl.-Chem., Lehrter
Strasse 52, D-3000 Hannover 73 (DE)**
Erfinder: **Bonse, Dirk, Dr. Dipl.-Ing., Krummer Kamp 3,
D-3160 Arpke / Lehrte (DE)**
Erfinder: **Meyer, Boris, Dr. Dipl.-Chem., Primasenser
Strasse 7, D-3000 Hannover 71 (DE)**

(74) Vertreter: **Lauer, Dieter, Dr., c/o Kali-Chemie AG
Postfach 220 Hans-Böckler-Allee 20,
D-3000 Hannover 1 (DE)**

**0 081 185**

## Beschreibung

Die Erfindung betrifft ein Verfahren zur Herstellung einer Glucose und Fructose enthaltenden Lösung durch Umsetzen einer Glucose enthaltenden Lösung an einem auf der Basis SiO$_2$-Träger hergestellten Katalysator mit Glucoseisomeraseaktivität.

Die enzymatische Umwandlung von Glucose in ein Glucose-Fructose-Gemisch hat in jüngster Zeit zunehmend an Bedeutung gewonnen. Das Gemisch wird dabei meist in Form eines Sirups, Isomerose genannt, vertrieben und ersetzt vor allem in der Lebensmittel- und Getränkeindustrie den weltweit knapper werdenden und teureren Kristallzucker (Rohrzucker, Rübenzucker, Saccharose) aus Zuckerrohr oder Zuckerrüben. Als Quelle für die zur Herstellung des Glucose-Fructose-Gemisches erforderliche Glucose dienen natürlich Stärkevorkommen, z. B. Mais- oder Kartoffelstärke, die durch saure und/oder enzymatische Hydrolyse in Glucose umgewandelt werden.

Zur enzymatischen Umwandlung der Glucose in die Fructose setzt man eine wäßrige Glucoselösung, gegebenenfalls unter Zusatz von die Isomerisierung begünstigenden Stoffen (z. B. Kobalt-, Magnesium-Ionen), der Einwirkung von Glucoseisomerase aus bis der gewünschte Isomerisierungsgrad erreicht ist, trennt dann gegebenenfalls die Zusätze von der Lösung ab und engt gegebenenfalls die Lösung zum Sirup ein.

In der Vergangenheit hat man dabei überwiegend batchweise gearbeitet und entweder isolierte Glucoseisomerase oder im natürlichen Zellverband des die Glucoseisomerase produzierenden Mikroorganismus belassene Glucoseisomerase benutzt. Da eine derart eingesetzte Glucoseisomerase nicht oder nur unter großem Aufwand rückgewinnbar und wiederverwendbar ist, wendet man in neuerer Zeit zunehmend durch Fixierung im Zellverband wasserunlöslich gemachte und durch Zusätze mechanisch verfestigte Glucoseisomerase an. Weiterhin kann Glucoseisomerase an anorganische oder organische Träger adsorptiv oder kovalent gebunden und dadurch wasserunlöslich gemacht werden. Eine derartig fixierte und verfestigte oder trägergebundene Glucoseisomerase kann mehrfach wiederverwendet werden und man kann damit zu kontinuierlichen Verfahren übergehen, beispielsweise indem man die trägergebundene Glucoseisomerase in einen Reaktor füllt und die Glucoselösung (Substrat) den Reaktor durchströmen läßt.

Die für solche kontinuierliche Verfahren benötigte trägergebundene Glucoseisomerase, nachfolgend Katalysator oder Trägerkatalysator mit Glucoseisomeraseaktivität genannt, ist an sich Stand der Technik. Als Beispiel hierfür sei die DE-PS 2 726 188 genannt, die insbesondere einen auf der Basis SiO$_2$-Träger hergestellten Katalysator mit Glucoseisomeraseaktivität beschreibt.

Das erfindungsgemäße Verfahren zur Herstellung einer Glucose und Fructose enthaltenden Lösung baut auf solchen kontinuierlich ausführbaren Verfahren auf, bei denen die Glucoselösung (das Substrat) einen Reaktor durchströmt, der einen Trägerkatalysator mit Glucoseisomeraseaktivität enthält.

Die Erfindung baut außerdem auf folgenden Erkenntnissen auf:

Die Konkurrenzfähigkeit der Isomerose wird im Vergleich zu natürlicher Saccharose neben ihrem Preis hauptsächlich von ihrem Fructose-Gehalt bestimmt, da dieser maßgeblich für deren Süßkraft ist. Im Gegensatz zur natürlichen Saccharose, einem Disaccarid aus Glucose und Fructose, in der Glucose und Fructose im Molverhältnis 1 : 1 vorliegen, ist der Gehalt an Glucose und Fructose in der Isomerose und damit deren Süßkraft schwankend und in erster Linie abhängig von der Zeit der Einwirkung der Glucoseisomerase auf die Glucoselösung und von der Temperatur, bei der die Einwirkung stattfindet. Das maximal bei 60°C erreichbare, nach hinreichend langer Zeit der Einwirkung sich einstellende thermodynamische Gleichgewicht liegt bei einem Isomerisierungsgrad von etwa 51%, d. h. von 100 eingesetzten Molekülen Glucose liegen 51 Moleküle als Fructose vor. Der Markt hat heute eine Isomerose mit einem Fructosegehalt von 42% in der Trockensubstanz akzeptiert. Da die Glucosegehalte der technischen Einsatzprodukte in der Regel zwischen 90 und 95% liegen, sind in der Praxis Isomerisierungsgrade von 44 bis 47% erforderlich, um diesen Fructosegehalt einzustellen. Um derartige Isomerisierungsgrade zu erzielen, können die Trägerkatalysatoren mit Glucoseisomeraseaktivität nur mit ganz bestimmten, von deren jeweiliger Aktivität abhängenden Raumgeschwindigkeit betrieben werden. Moderne, hochaktive Katalysatoren, wie sie beispielsweise nach dem Verfahren der DE-PS 2 726 188 herstellbar sind, können mit Anfangsraumgeschwindigkeiten von etwa 10 bis 20 v/vh betrieben werden.

Mit zunehmender Betriebszeit sinkt jedoch die Aktivität der bekannten Katalysatoren in Abhängigkeit von der Reaktionstemperatur mehr oder weniger schnell ab.

Der bekannte Katalysator nach der DE-PS 2 726 188 besitzt nach etwa 670 Betriebsstunden bei 60°C Reaktionstemperatur noch 50% seiner Anfangsaktivität. Um denselben Isomerisierungsgrad wie zu Beginn zu erhalten, kann ein solcher Katalysator nur noch mit der halben Anfangsraumgeschwindigkeit betrieben werden. 20% Restaktivität, die als untere Grenze der Wirtschaftlichkeit anzusehen sind, werden nach 1700 Betriebsstunden erreicht.

Überraschenderweise wurde nun gefunden, daß die Betriebszeit eines auf Basis SiO$_2$-Träger hergestellten Katalysators mit Glucoseisomeraseaktivität mehr als verdoppelt werden kann, wenn man die Glucose enthaltende Lösung vor der Umsetzung an dem Trägerkatalysator mit Formlingen aus SiO$_2$ oder Alumosilikat in Berührung bringt. Das Massenverhältnis Katalysator : Formlinge bewegt sich zweckmäßigerweise zwischen 3 : 1 und 1 : 3, vorzugsweise bei 1 : 1. Bei dieser Verfahrensweise ver-

2

doppelt sich auch die Produktivität des Katalysators. Die Produktivität ist definiert als die Substratmenge, berechnet als Trockensubstanz [kg], die bei einem gegebenen Isomerisierungsgrad von 1 kg Katalysator bis zu einer Restaktivität von 20% der Anfangsaktivität verarbeitet werden kann.

Bei einer Anlage zur insbesondere kontinuierlichen Umwandlung der Glucose in die Fructose, welche neben dem Reaktor für den Trägerkatalysator mit Glucoseisomeraseaktivität die sonst noch erforderlichen Bestandteile wie Vorrats- und Auffangbehälter, Leitungen, Pumpen, Meßgeräte, Temperiereinrichtungen und dgl. enthält, ist erfindungsgemäß dem Reaktor eine Vorsäule vorgeschaltet, die der Aufnahme der Formlinge aus SiO$_2$ oder Alumosilikat dient. Das Volumen der Vorsäule ist dabei im Verhältnis zum Volumen des Reaktors so gehalten, daß unter Berücksichtigung der Schüttgewichte von Katalysator und Vorsäulenmaterial die obengenannten Massenverhältnisse gewahrt werden können.

Die Ausführung des erfindungsgemäßen Verfahrens erfolgt bei insbesondere kontinuierlicher Arbeitsweise derart, daß man eine wäßrige Glucoselösung, vorzugsweise mit einem Gehalt von etwa 40 bis 50 Gew.-% Trockensubstanz, auf einen für die trägergebundene Glucoseisomerase geeigneten pH-Wert einstellt, die Lösung auf die geeignete Isomerisierungstemperatur erwärmt und die Lösung zunächst durch die mit SiO$_2$ oder Alumosilikat gefüllte Vorsäule und dann durch die mit dem SiO$_2$-Trägerkatalysator mit Glucoseisomeraseaktivität gefüllten Reaktor pumpt. Die ablaufende Glucose-Fructose-Lösung wird in regelmäßigen Abständen auf ihren Fructose-Gehalt analysiert, beispielsweise mittels polarimetrischer Analyse oder HPLC. Die Raumgeschwindigkeit, mit der der Katalysator betrieben wird, wird dabei so eingestellt, daß die ablaufende Lösung, bezogen auf Trockensubstanz, einen Gehalt von etwa 42 Gew.-% Fructose aufweist.

Für das Vorsäulenmaterial hat sich eine Korngröße von etwa 0,5 bis 5,0 mm und für den Trägerkatalysator eine Korngröße von etwa 0,08 bis 0,5 mm als besonders vorteilhaft erwiesen.

Falls bei der Herstellung des Trägerkatalysators mit Glucoseisomeraseaktivität eine Streptomyces albus Glucoseisomerase verwendet wurde, hat sich die Einstellung der Glucoselösung auf einen pH von etwa 7,0 bis 8,5 und eine Erwärmung der Glucoselösung auf eine Temperatur von etwa 55 bis 65° C als besonders günstig erwiesen.

Außerdem hat sich bei einer Streptomyces albus Glucoseisomerase die Zugabe von Co(II)- und Mg(II)-Ionen zur Glucoselösung in Mengen von etwa 0,1 bis 2 ppm Co(II) und etwa 10 bis 200 ppm Mg(II), zweckmäßigerweise in Form ihrer wasserlöslichen Salze wie beispielswiese Chloride oder Sulfate, als isomerisierungsfördernd erwiesen.

Schließlich ist es noch vorteilhaft, der Glucoselösung eine stabilisierende Menge eines Antioxidants, vorzugsweise SO$_2$ in einer Menge von etwa 100 bis 600 ppm in Form von Alkalimetallsulfit oder -bisulfit, hinzuzufügen.

Bevor die erhaltene Glucose-Fructose-Lösung zum Sirup eingeengt oder die Lösung ihrer endgültigen Verwendung zugeführt wird, ist es noch empfehlenswert, die unerwünschten, weil beispielsweise geschmacksbeeinträchtigenden ionischen Komponenten mittels Kationen- und Anionenaustauschern aus der Glucose-Fructose-Lösung zu entfernen.

Die Erfindung soll anhand der folgenden Beispiele erläutert werden:

## Beispiel 1

5 g eines Trägerkatalysators mit Glucoseisomeraseaktivität, hergestellt gemäß der DE-PS 2 726 188, und mit den nachstehend beschriebenen Eigenschaften wurden in einen Reaktor gefüllt, dem eine Säule vorgeschaltet war, die 5 g eines kommerziell erhältlichen kugelförmigen, wasserfesten, porösen Alumosilikats (z. B. Typ KCT-WS der Kali-Chemie AG, Zusammensetzung etwa 97 Gew.-% SiO$_2$ und 3 Gew. -% Al$_2$O$_3$) enthielt. Durch dieses System Vorsäule-Reaktor wurde eine auf 60° C erwärmte Glucoselösung gepumpt. Die Einstellung der Raumgeschwindigkeit (bezogen auf das vom Katalysator in Anspruch genommene Reaktorvolumen) erfolgt so, daß der Isomerisierungsgrad über die gesamte Betriebszeit konstant 46,5% betrug. Der Isomerisierungsgrad der ausfließenden Substratlösung wurde polarimetrisch gemessen. Im einzelnen sind Katalysator, Vorsäulenfüllung und Verfahren durch folgende Parameter gekennzeichnet:

### 1. Katalysator

| | | |
|---|---|---|
| 1.1 | Träger | SiO$_2$ |
| 1.2 | Aktivitätsaufnahme | 9000 U/g |
| 1.3 | Korngröße | 0,1—0,2 mm |
| 1.4 | Schüttgewicht (trocken) | 0,45 kg/l |

3

## 2. Vorsäulenfüllung

| | | |
|---|---|---|
| 2.1 | Füllmaterial | KCT-WS (Kali-Chemie AG) |
| 2.2 | Korngröße | 1—2 mm |
| 2.3 | Schüttgewicht (trocken) | 0,70 kg/l |

## 3. Verfahren

| | | |
|---|---|---|
| 3.1 | Substrat | 45 Gew.-% Glucose in wäßriger Lösung |
| 3.2 | Kofaktoren | 120 ppm Mg (II) |
| | | 1 ppm Co (II) |
| | | 200 ppm $SO_2$ (in Form von $Na_2SO_3$) |
| 3.3 | pH-Wert | 7,5 |
| 3.4 | Substratdichte | 1,2 kg/l |
| 3.5 | Substrateingangstemperatur | 60°C |
| 3.6 | Isomerisierungsgrad | 46,5% |
| 3.7 | Anfangsraumgeschwindigkeit | 13,0 h$^{-1}$ |

## 4. Aktivitätsbestimmung der Glucoseisomeraselösung

Die Aktivität der zur Präparation des Katalysators eingesetzten Glucoseisomeraselösung wurde nach der Takasaki Methode (vgl. Y. Takasaki: Agr. Biol. Chem. Vol. 30, Nr. 12, 1247—1253, 1966 und Z. Dische und E. Borenfreund: J.-Biol. Chem. 192, 583, 1951) bestimmt. Eine Aktivitätseinheit (U) ist definiert als die Enzymemenge, die unter Inkubationsbedingungen 1 mg Fructose bildet.

| | |
|---|---|
| Inkubationsbedingungen | |
| Temperatur | 65°C |
| Reaktionszeit | 1 h |
| Substrat | 0,1 m Glucose × $H_2O$ (Merck 8342) in 0,05 m Phosphatpuffer, pH 8,0 mit 0,0004 m $MgSO_4$ |

Das Beispiel brachte folgende Ergebnisse

| | |
|---|---|
| Halbwertszeit | 1300 h |
| Betriebszeit bis Restaktivität 20% | 3800 h |
| Mittlere Aktivität über Betriebszeit 3800 h | 44,0% (bezogen auf Anfangsaktivität 100%) |
| Produktivität nach 3800 h | 26 000 kg TS mit 46,5% Fructose/kg Katalysator |

## Beispiel 2

5 g eines Trägerkatalysators entsprechend Beispiel 1 wurden in einen Reaktor gefüllt, dem eine Säule vorgeschaltet war, die 10 g eines kommerziell erhältlichen kugelförmigen, wasserfesten, porösen $SiO_2$ (z. B. Typ AF 125 der Kali-Chemie AG, $SiO_2$-Gehalt größer 99 Gew.-%) enthielt. Die weitere Verfahrensweise entsprach Beispiel 1. Die Vorsäulenfüllung war durch folgende Parameter gekennzeichnet:

| | | |
|---|---|---|
| 1. | Füllmaterial | AF 125 (Kali-Chemie AG) |
| 2. | Korngröße | 1—2 mm |
| 3. | Schüttgewicht (trocken) | 0,45 kg/l |

Die Versuchsergebnisse waren mit den im Beispiel 1 beschriebenen identisch.

**0 081 185**

Beispiel 3

Zum Vergleich wurde Beispiel 1 mit dem gleichen Katalysator, jedoch ohne Vorsäule, aber unter sonst gleichen Verfahrensbedingungen wiederholt. Beispiel 3 brachte folgende Ergebnisse

| | |
|---|---|
| Halbwertszeit | 670 h |
| Betriebszeit bis | |
| Restaktivität 20% | 1700 h |
| Mittlere Aktivität über | |
| Betriebszeit 1700 h | 47,2% (bezogen auf Anfangsaktivität 100%) |
| Produktivität nach 1700 h | 12 500 kg TS mit 46,5% Fructose/kg Katalysator |

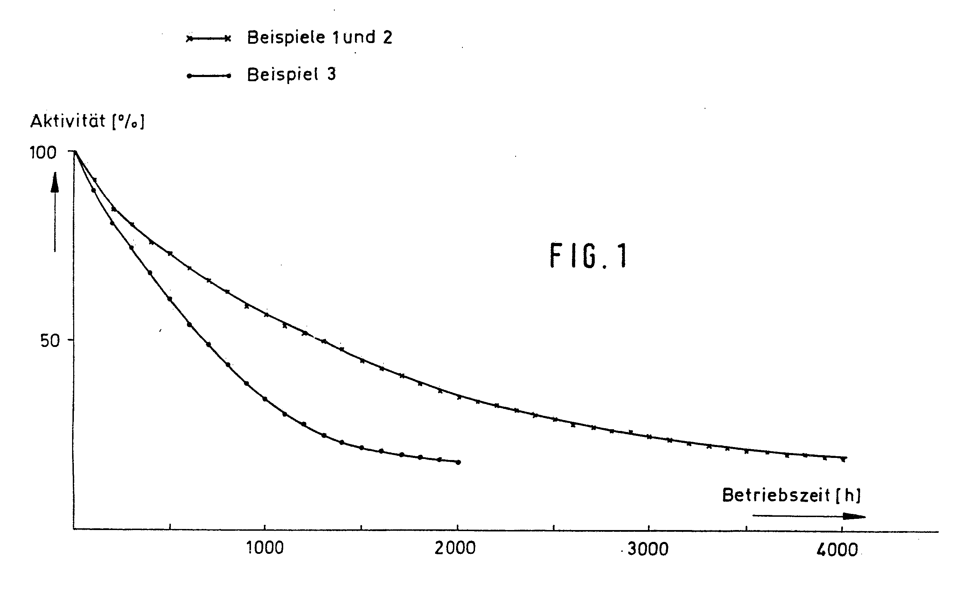

Zur Veranschaulichung der Ergebnisse gemäß den Beispielen 1, 2 und 3 wurde die Aktivitätsabnahme in Abhängigkeit von der Betriebszeit in Abb. 1 dargestellt. In dieser Darstellung wird die durch erfindungsgemäße Anwendung der mit Alumosilikat- oder $SiO_2$-Perlen gefüllten Vorsäule (Beispiele 1, 2) erhaltene Stabilisierung der Aktivität des Katalysators über eine lange Betriebsperiode deutlich.

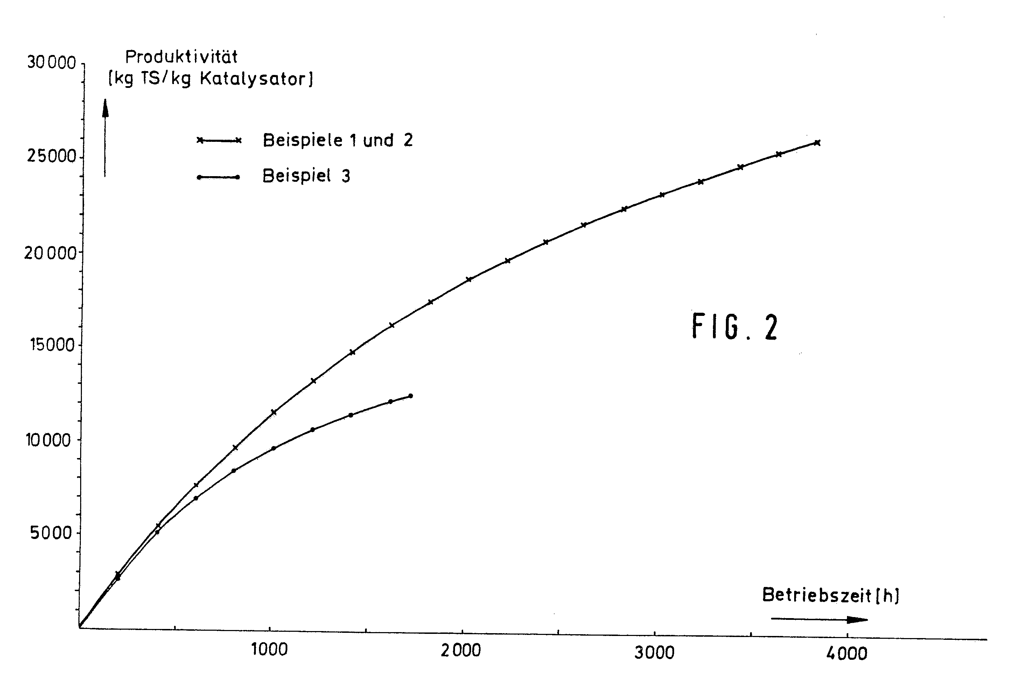

In Abb. 2 ist die für die Wirtschaftlichkeit des Verfahrens entscheidende Entwicklung der Produktivität in Abhängigkeit von der Betriebszeit dargestellt. Die unter den erfindungsgemäßen Verfahrensbedingungen durchgeführten Beispiele 1 und 2 erhöhten die wirtschaftlich nutzbare spezifische Leistungsfähigkeit des Katalysators um den Faktor 2.08, d. h. zur Herstellung einer bestimmten Menge Isomerose wird nur noch weniger als die halbe Katalysatormenge benötigt. Da das erfindungsgemäß benötigte Vorsäulenmaterial nur einen Bruchteil der Katalysatorkosten ausmacht, ist der wirtschaftliche Vorteil des erfindungsgemäßen Verfahrens erheblich.

**Patentansprüche**

1. Verfahren zur Herstellung einer Glucose und Fructose enthaltenden Lösung durch Umsetzen einer Glucose enthaltenden Lösung an einem auf der Basis $SiO_2$-Träger hergestellten Katalysator mit Glucoseisomeraseaktivität, dadurch gekennzeichnet, daß man die Glucose enthaltende Lösung vor der Umsetzung an dem Trägerkatalysator mit Formlingen aus $SiO_2$ oder Alumosilikat in Berührung bringt.

2. Verfahren nach Anspruch 1, dadurch gekennzeichnet, daß das Masseverhältnis Katalysator : Formlinge zwischen 3 : 1 und 1 : 3, vorzugsweise bei 1 : 1 liegt.

3. Anlage zur Ausführung des Verfahrens nach einem der Ansprüche 1 oder 2 mit einem mit dem Trägerkatalysator gefüllten Reaktor, dadurch gekennzeichnet, daß dem Reaktor eine mit Formlingen aus $SiO_2$ oder Alumosilikat gefüllte Vorsäule vorgeschaltet ist.

**Claims**

1. Process for the preparation of a solution containing glucose and fructose by reacting a glucose-containing solution over a catalyst which has glucose isomerase activity and is prepared on the basis of an $SiO_2$ support, characterised in that the glucose-containing solution is brought into contact with shaped pieces of $SiO_2$ or alumosilicate before the reaction on the supported catalyst.

2. Process according to claim 1, characterised in that the weight ratio of catalyst to shaped pieces is between 3 : 1 and 1 : 3, preferably 1 : 1.

3. Apparatus for carrying out the process according to one of claims 1 or 2 with a reactor filled with the supported catalyst, characterised in that the reactor is connected with a preliminary column filled with shaped pieces of $SiO_2$ or alumosillicate.

**Revendications**

1. Procédé de préparation d'une solution contenant du glucose et du fructose par réaction d'une solution contenant du glucose sur un catalyseur préparé à base d'un support de $SiO_2$ et présentant de l'activité de glucose isomérase, procédé caractérisé en ce que, avant la réaction sur le catalyseur sur support, on met la solution contenant le glucose en contact avec des objets façonnés en $SiO_2$ ou en aluminosilicate.

2. Procédé selon la revendication 1, caractérisé en ce que le rapport pondéral entre le catalyseur et les objets façonnés se situe entre 3 : 1 et 1 : 3 et est avantageusement égal à 1 : 1.

3. Installation pour la mise en oeuvre cu procédé selon l'une des revendications 1 ou 2, comportant un réacteur garni du catalyseur sur support, installation caractérisée en ce que, en amont du réacteur, est disposée une colonne préliminaire garnie d'objets façonnés en $SiO_2$ ou en aluminosilicate.

5

FIG. 1

FIG. 2

Produktivität
[kg TS/kg Katalysator]

×——× Beispiele 1 und 2

•——• Beispiel 3

Betriebszeit [h]